# EUROPÄISCHE PATENTSCHRIFT

(11) **EP 2 164 527 B1**
(45) Veröffentlichungstag und Bekanntmachung des Hinweises auf die Patenterteilung: **01.05.2013**
(21) Anmeldenummer: 08774278.9
(22) Anmeldetag: 25.06.2008
(51) Int. Cl.: A61L 2/08, B65B 55/08, B67C 7/00

(54) **STERILISATION MIT ß-STRAHLUNG**
STERILIZATION WITH ß-RADIATION
STÉRILISATION PAR RAYONNEMENT ß

(30) Priorität: 26.06.2007 DE 102007029567
(43) Veröffentlichungstag der Anmeldung: 24.03.2010
(73) Patentinhaber: Krones AG, 93073 Neutraubling (DE)
(72) Erfinder: ZIEGLER, Manfred, 94034 Passau (DE); KRÜGER, Jochen, 93095 Hagelstadt (DE)
(74) Vertreter: Hannke, Christian
(86) Internationale Anmeldenummer: PCT/EP2008/058070
(87) Internationale Veröffentlichungsnummer: WO 2009/000850

(56) Entgegenhaltungen:
- EP-A- 0 340 411
- WO-A-2004/000100
- WO-A-2005/108278
- FR-A- 2 255 916
- US-A- 3 780 308

## Beschreibung

Die vorliegende Erfindung bezieht sich auf eine Vorrichtung zum Befüllen von Behältnissen. In der Getränkeindustrie sind diverse Vorrichtungen zum Befüllen von Behältnissen, die im Folgenden auch als Füller bezeichnet werden, seit langem bekannt. Insbesondere bei der Befüllung der Behältnisse mit Lebensmitteln, wie Säften, Wasser und dergleichen, sind dabei von den Abfüllern strenge Sicherheitsmaßnahmen zu beachten. So ist es erforderlich, dass die Behältnisse vor dem eigentlichen Befüllungsvorgang sterilisiert werden. Weiterhin ist darauf zu achten, dass die Behältnisse auch während des eigentlichen Befüllungsvorganges nicht verunreinigt werden.

Vorrichtungen und Verfahren auf dem technischen Gebiet der Elektronenstrahlsterilisation sind aus den Druckschriften US3780308, WO2004000100, EP0340411, FR2255916, WO2005108278 bekannt.

Der vorliegenden Erfindung liegt daher die Aufgabe zugrunde, eine Vorrichtung und ein Verfahren zum Befüllen von Behältnissen zur Verfügung zu stellen, welche einen hohen Sterilisationsgrad der angefüllten Behältnisse erlauben. Dies wird durch eine Vorrichtung nach Anspruch 1, sowie ein Verfahren zum Behandeln von Behältnissen nach Anspruch 10 erreicht. Vorteilhafte Ausführungsformen und Weiterbildungen sind Gegenstand der Unteransprüche.

Eine erfindungsgemäße Vorrichtung zum Befüllen von Behältnissen weist einen Zugang auf, durch welchen hindurch offene Behältnisse in die Vorrichtung einführbar sind. Weiterhin ist eine Transporteinrichtung vorgesehen, welche die Behältnisse stromabwärts bezüglich des Zugangs entlang des vorgegebenen Transportpfads befördert und weiterhin ist ein Abgang vorgesehen, über den die Behältnisse aus der Vorrichtung abführbar sind. Dabei ist die Geschwindigkeit, mit der die Transporteinrichtung die Behältnisse befördert, veränderbar und es ist weiterhin wenigstens eine Befüllungseinrichtung vorgesehen, welche die Behältnisse auf den Transportpfad zwischen dem Zugang und dem Abgang wenigstens teilweise mit einem flüssigen Medium befüllt.

Erfindungsgemäß weist die Vorrichtung ein Gehäuse auf und im Inneren dieses Gehäuses ist wenigstens eine Sterilisationseinrichtung im Form einer ß-Strahlungseinheit und etwa auch Röntgenstrahlung angeordnet, welche Komponenten der Vorrichtung sterilisiert. Vorzugsweise handelt es sich bei dem Gehäuse um ein geschlossenes Gehäuse, so dass verhindert wird, dass ß-Strahlung und etwa auch Röntgenstrahlung aus der Vorrichtung austritt. Bei der Transporteinrichtung handelt es sich besonders bevorzugt um ein Transportkarussell, auf dem eine Vielzahl von Befüllungseinrichtungen angeordnet ist, welche die Behältnisse mit dem Getränk befüllen. Weiterhin sind vorzugsweise innerhalb der Vorrichtung noch mehrere Transporteinrichtungen, beispielsweise in Form von Übergabesternen oder dergleichen angeordnet. Die β-Strahlungseinheit bzw. der ß-Strahler bewirkt durch die Ausstrahlung von ß-Strahlung eine Sterilisation von Komponenten der Vorrichtung und bewirkt auf diese Weise insbesondere auch im laufenden Betrieb eine Sterilhaltung der Vorrichtung.

Die Intensität dieser wenigstens einen β-Strahlungseinheit ist steuerbar. Unter steuerbar wird dabei verstanden, dass die Strahlungseinheit nicht nur zwischen einem eingeschalteten und einem ausgeschalteten Zustand schaltbar ist, sondern in wenigstens zwei verschiedenen Intensitätsstufen und bevorzugt stufenlos zwischen einen minimalen Intensitätswert und einen maximalen Intensitätswert steuerbar ist. So ist es beispielsweise möglich, die Beschleunigungsspannung in einer Beschleunigungsröhre der β-Strahlungseinheit zu verändern, um die Intensität der β-Strahlung zu variieren.

Bei einer bevorzugten Ausführungsform weist die Vorrichtung eine Steuereinheit auf, die derart gestaltet ist, dass sie die Intensität der wenigstens einen ß-Strahlungseinheit in Abhängigkeit von einem Betriebsparameter der Vorrichtung steuert. Es handelt sich bei dem Betriebsparameter um die Transportgeschwindigkeit der Transporteinrichtung. Allgemein ist eine bestimmte Dosis an ß-Strahlung nötig, um eine effiziente Sterilisation der jeweiligen Komponenten zu erreichen. Falls die Maschinengeschwindigkeit gedrosselt wird, befinden sich die einzelnen Komponenten über einen längeren Zeitraum hinweg in dem Einstrahlungsbereich einer bestimmten β-Strahlungseinheit. In diesem Falle wird damit bevorzugt die Intensität der betreffenden ß-Strahlungseinheit reduziert. Auf diese Weise kann eine geforderte Dosis an ß-Strahlung, die nach Möglichkeit weder unterschritten noch überschritten werden sollte, auch bei unterschiedlichen Transportgeschwindigkeiten erreicht werden.

Erfindungsgemäβ handelt es sich bei dem Betriebsparameter der Vorrichtung um einen Öffnungszustand einer stromaufwärts bezüglich der Vorrichtung vorgesehen Sperreinrichtung für die Behältnisse. Beispielsweise kann es im Fall von Ausfällen anderer Einheiten einer kompletten Anlage dazu kommen, dass der Strom der Behältnisse unterbrochen werden muss. Falls eine derartige Sperreinrichtung geschlossen ist und damit keine Behältnisse mehr in die Vorrichtung gelangen, wird bei der bevorzugten Ausführungsform, sobald sich keine Behältnisse mehr in der Vorrichtung befinden, die entsprechende Strahlungsleistung reduziert und damit ist es möglich, dass die erforderliche Kühlleistung für die ß-Strahlungseinheit ebenfalls reduziert werden kann. Dabei bewirkt die Steuerungseinrichtung jedoch, dass ein Absenken der Intensität bzw. Strahlungsleistung erst erfolgt, wenn sich keine Behältnisse mehr innerhalb der Vorrichtung befinden. Auf diese Weise wird ein ungleichmäßiges Sterilisieren bestimmter Komponenten, was sich wiederum auf die Behältnisse selbst auswirken kann, verhindert.

Bei einer bevorzugten Ausführungsform sind die von der β-Strahlungseinheit bestrahlten Komponenten aus einer Gruppe von Komponenten ausgewählt, welche Transportsterne, Garnituren, Greifeinrichtungen für die Behältnisse aufweist. Es wird damit darauf hingewiesen, dass durch die erfindungsgemäße Vorrichtung nicht die Behältnisse selbst mit ß-Strahlen bestrahlt werden, sondern lediglich die Komponenten, welche die Behältnisse behandeln.

Bei einer weiteren vorteilhaften Ausführungsform ist die wenigstens eine ß-Strahlungseinheit derart beschaffen und/oder angeordnet, dass der aus der ß-Strahlungseinheit austretende ß-Strahl während des Transports der Behältnisse nicht auf die Behältnisse gelenkt wird. Es kann beispielsweise ein Abdeckblech vorgesehen sein, welches über den transportierten Behältnissen angeordnet ist und die Strahlungseinheit sterilisiert lediglich Bereiche oberhalb dieses Abdeckblechs. Weiterhin können die ß-Bestrahlungseinheiten so angeordnet sein, dass eine Sterilisierung unterhalb des Transportpfades der Behältnisse und insbesondere auch unterhalb der Behältnisse selbst stattfindet.

Weiterhin können die ß-Strahlungseinheiten an Bereichen der Transporteinrichtung vorgesehen sein, an denen sich zu keinem Zeitpunkt Behältnisse befinden. Werden beispielsweise als Transporteinrichtung Transportsterne verwendet, so ist üblicherweise stromaufwärts bezüglich dieser Transportsterne ein Übergabestern vorgesehen, der die Behältnisse an den Transportstern übergibt sowie stromabwärts bezüglich des Transportsterns ein Übernahmestern, welcher die Behältnisse von dem Transportstern aufnimmt. In Umfangsrichtung des Transportsterns ist üblicherweise ein gewisser Winkelbereich vorgesehen, in dem die Greifeinrichtungen für die Behältnisse stets leer sind, das heißt in dem sich zu keinem Zeitpunkt Behältnisse befinden. Bei einer bevorzugten Ausführungsform werden die ß-Strahlungseinheiten in einem solchen Bereich angeordnet bzw. so angeordnet, dass lediglich ein solcher Bereich bestrahlt wird. Manche Lebensmittelrichtlinien, wie beispielsweise Richtlinien der US-amerikanischen FDA (Food and Drugs Administration) schreiben vor, dass Strahlen, wie hier ß-Strahlen, nicht mit dem eigentlichen Getränk in Berührung kommen dürfen, da eine Kontamination des Getränks möglich ist. Dies gilt dabei auch für Tropfen des Getränks, welche noch in die Behältnisse gelangen können.

Jedoch kann auch durch spezielle Ausführungsformen der β-Strahlungseinheiten bewirkt werden, dass das abgegebene Strahlungsprofil nicht mit den Behältnissen in Kontakt kommt. So können durch speziell angepasste Blenden der β-Strahlungseinheit geometrisch genau festgelegte "Strahlungskeulen" erzeugt werden. Dabei ist zu berücksichtigen, dass die ß-Strahlung durch das Medium, beispielsweise sterilisierte Luft oder auch Stickstoff oder ein Edelgas abgeschwächt wird, und damit unter Berücksichtigung auch dieser Komponenten sowie der Anordnung der Sterilisationseinrichtung selbst ein genaues Profil dieser "Strahlungskeule" vorgegeben werden kann.

Bei einer weiteren bevorzugten Ausführungsform ist die Vorrichtung in einem Reinraum angeordnet und dieser Reinraum ist mit einem Gas gefüllt, welches aus einer Gruppe von Gasen ausgewählt ist, welche Edelgase, Stickstoff, sterilisierte Luft enthält. Vorzugsweise steht dabei das erwähnte Gas in dem die Vorrichtung umgebenden Raum unter einem überatmosphärischen Druck.

Bei einer weiteren bevorzugten Ausführungsform ist eine Vielzahl von stationär angeordneten ß-Strahlungseinheiten vorgesehen. Durch das Vorsehen von stationär angeordneten Strahlungseinheiten kann in besonders vorteilhafter Weise erreicht werden, dass die Behältnisse nicht mit der β-Strahlung bzw. dem Elektronenstrahl in Berührung kommen.

Die vorliegende Erfindung ist weiterhin auf eine Anlage zum Behandeln von Behältnissen mit einer Vorrichtung der oben beschriebenen Art und einer stromaufwärts bezüglich dieser Vorrichtung angeordneten Sterilisationseinrichtung, welche die Behältnisse sterilisiert, gerichtet. Dabei wird darauf hingewiesen, dass in der genannten Sterilisationseinrichtung die Behältnisse selbst innen und außen sterilisiert werden und damit vollständig sterilisiert in der Vorrichtung ankommen. Die Begriffe stromaufwärts und stromabwärts werden dabei unter Bezugnahme auf die Transportrichtung der Behältnisse festgelegt.

Dabei weist bevorzugt die Sterilisationseinrichtung wenigstens eine Fördereinrichtung auf, welche die Behältnisse durch die Sterilisationseinrichtung fördert.

Vorzugsweise sind die Antriebe der Transporteinrichtung und der Fördereinrichtung unabhängig voneinander steuerbar. Damit ist es möglich, die Transporteinrichtung und die Fördereinrichtung unabhängig voneinander zu betreiben. Grundsätzlich ist es im Stand der Technik üblich, mehrere Antriebe aufeinander zu synchronisieren und damit einen Blockbetrieb zu ermöglichen. Bei dieser bevorzugten Ausführungsform wird nun diese Synchronisation zumindest zeitweise aufgehoben. Dieses Aufheben der Synchronisation ist, wie unten genauer erläutert wird, insbesondere für Anfahrprozesse interessant. Vorzugsweise werden für die Fördereinrichtung (der Sterilisationseinrichtung) und die Transporteinrichtung separate Antriebe und besonders bevorzugt Antriebe in Form von Servomotoren vorgesehen.

Bevorzugt ist es jedoch auch möglich, den Antrieb der Transporteinrichtung zeitweise auf den Antrieb der Fördereinrichtung zu synchronisieren. Dies ist insbesondere für den normalen Produktionsbetrieb interessant.

Die vorliegende Erfindung ist weiterhin auf ein Verfahren zum Behandeln von Behältnissen gerichtet. Dabei werden in einem ersten Verfahrensschritt geöffnete Behältnisse einer Vorrichtung zum Befüllen von Behältnissen und insbesondere eine Vorrichtung der oben beschriebenen Art zugeführt. In einem Verfahrensschritt werden die Behältnisse transportiert und während dieses Transports befüllt. Schließlich werden die gefüllten, jedoch bevorzugt noch geöffneten Behältnisse, aus der Vorrichtung zum Befüllen von Behältnissen abgeführt.

Erfindungsgemäß werden die Komponenten der Vorrichtung zum Befüllen von Behältnissen während des Befüllungsvorgangs durch wenigstens eine Sterilisationseinrichtung sterilisiert und diese Sterilisationseinrichtung weist wenigstens eine β-Strahlungseinheit auf. Bevorzugt werden eine oder mehrere Komponenten der Vorrichtung durch eine Vielzahl von β-Strahlungseinheiten sterilisiert.

Vorzugsweise wird die Intensität der ß-Strahlungseinheiten in Abhängigkeit von einer Transportgeschwindigkeit der Behältnisse durch die Vorrichtung gesteuert. Im Falle einer niedrigen Transportgeschwindigkeit kann entsprechend die Leistung der Strahlungseinheit herabgesetzt werden, um eine nötige bestimmte Dosis einzuhalten bzw. zu erreichen.

Vorzugsweise wird die Vorrichtung wenigstens zeitweise in einem Leerlaufmodus betrieben, in dem keine Behältnisse durch die Vorrichtung transportiert werden, jedoch die Transportgeschwindigkeit der Vorrichtung von Null verschieden ist, wobei in diesem Leerlaufmodus die Intensität wenigstens einer ß-Strahlungseinheit reduziert wird. Es wird dabei darauf hingewiesen, dass auch in einem Leerlaufmodus, in dem sich keine Behältnisse in der Vorrichtung befinden, vorzugsweise eine bestimmte Geschwindigkeit der Komponenten, beispielsweise der Greifeinrichtungen oder der Transportsterne, beibehalten wird, um sicherzustellen, dass die Sterilisation auch während dieses Ruhebetriebs weiter erfolgt. Auf diese Weise kann ein hoher Sterilisationsgrad der gesamten Vorrichtung auch in Ruhezeiten beibehalten werden.

Bevorzugt werden die Behältnisse vor dem Befüllen mit einer Sterilisationseinrichtung sterilisiert. Dabei ist es besonders bevorzugt möglich, dass im Rahmen eines Anfahrmodus die Fördergeschwindigkeit, mit der die Sterilisationseinrichtung die Behältnisse befördert und die Transportgeschwindigkeit, mit der die Vorrichtung die Behältnisse transportiert aufeinander angepasst werden. So ist es beispielsweise möglich, dass die Transporteinrichtung der Vorrichtung im Rahmen eines Synchronisationsvorgangs auf die Geschwindigkeit Fördereinrichtung der Sterilisationseinrichtung sterilisiert wird. Damit wird in diesem Synchronisationsprozess die Blockung zu der genannten Sterilisationseinrichtung durchgeführt, wobei dies bevorzugt mittels Servotechnik erfolgt. Diese Blockung wird dabei in dem Modus "on-the fly" ausgeführt. Dadurch ist es möglich, dass beispielsweise bei Produktionsbeginn eine strahlerbedingte Geschwindigkeitsabsenkung bzw. ein sogenanntes Erstrundenanfahren erfolgt. Auch ist es durch diesen Synchronisationsbetrieb möglich, eine mögliche Produktentleerung oder dergleichen durchzuführen.

Bevorzugt erfolgt die Anpassung der Geschwindigkeit der Transporteinrichtung und der Förderungseinrichtung während einer vorgegebenen Zeitspanne und diese Zeitspanne entspricht im Wesentlichen einem ganzteiligen Vielfachen derjenigen Zeitspanne, in der die Behältnisse in einer kompletten Umdrehung in der Transporteinrichtung befördert würden. Falls als Transporteinrichtung beispielsweise ein Transportkarussell verwendet wird, findet die Anpassung in demjenigen Zeitraum statt, in dem ein bestimmter Greifarm im Wesentlichen eine, zwei, drei ... oder n-Umdrehungen durchführt. Auf diese Weise wird sichergestellt, dass es nicht zu ungleichmäßigen Sterilisationen während dieses Beschleunigungs- oder Anpassungsvorgangs kommt. Besonders bevorzugt wird die Geschwindigkeit der Transporteinrichtung linear angepasst, das heißt der Beschleunigungs- oder Verzögerungsvorgang erfolgt linear. Dadurch, dass die Transporteinrichtung und damit auch die auf diese synchronisierten weiteren Transportsterne während des Anfahrvorgangs langsamer fahren, kann eine höhere Dosis an β-Strahlung auf diese aufgebracht werden. Dadurch, dass die Verzögerung während eines bestimmten Zeitraums vorgenommen wird, wird erreicht, dass die Sterilisation einheitlich für alle Füllstationen erfolgt. Auf diese Weise kann letztlich erreicht werden, dass die Menge an Ausschussbehältnissen durch die genannten Anfahrprozesse gering gehalten wird.

Weitere Vorteile und Ausführungsformen ergeben sich aus den beigefügten Zeichnungen:
Darin zeigen:
   - Fig. 1: eine schematische Darstellung einer erfindungsgemäßen Vorrichtung; und
   - Fig. 2: ein Zeit - Geschwindigkeits - Diagramm für eine erfindungsgemäße Vorrichtung.

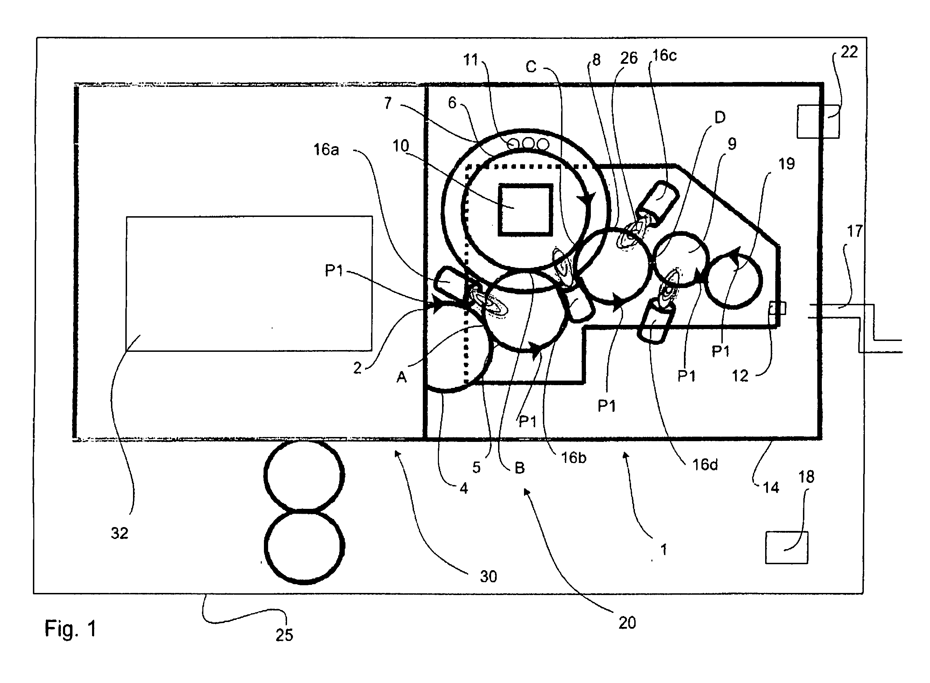

Fig. 1 zeigt eine schematische Darstellung einer erfindungsgemäßen Anlage 20 zum Behandeln von Behältnissen. Diese Anlage 20 weist eine Vorrichtung 1 zum Befüllen von Behältnissen bzw. einen Füller 1 auf. Dabei werden die (nicht gezeigten) Behältnisse durch den Füller 1 von einem Zugang 2 jeweils entlang der mit P1 gekennzeichneten Pfeile in Richtung eines Abgangs 12 transportiert. Beim Transport der Behältnisse wird eine Vielzahl von Transportkarussells 4, 5, 8, 9, 19 verwendet. Bei diesen Transportkarussells sind jeweils (nicht gezeigte) Greifeinrichtungen vorgesehen, welche die Behältnisse an das jeweils nächste Karussell übergeben. An die Vorrichtung 1 schließt sich ein Labyrinthpfad 17 an, durch den verhindert wird, dass Strahlung aus der Vorrichtung 1 austreten kann. Weiterhin schließt sich an die Vorrichtung 1 eine (nicht gezeigte) Verschließeinrichtung an, welche die Behältnisse nach deren Befüllung verschließt. Damit gelangen die unverschlossenen Behältnisse in den Füller 1, werden dort befüllt und werden anschließend in gefülltem, jedoch noch geöffnetem Zustand wieder aus dem Füller herausgegeben. Damit müssen die einzelnen Transporteinrichtungen 4, 5, 8, 18, 19 beispielsweise hinsichtlich ihrer Bewegungen auf den Transport von unverschlossenen, gefüllten Behältnissen angepasst sein.

Das Bezugszeichen 10 zeigt schematisch die eigentliche Befüllungseinrichtung, welche die Behältnisse befüllt. Diese Befüllungseinrichtung 10 weist eine Vielzahl von nur schematisch dargestellten Befüllungselementen 11 auf, die auf einem Karussell 6 angeordnet sind. Dieses Karussell 6 ist damit die oben erwähnte Transporteinrichtung 6. Bei diesen Befüllungselementen 11 kann es sich beispielsweise um Düsen handeln, die das Getränk in die Behältnisse einfüllen. Das Bezugszeichen 7 kennzeichnet eine Abdeckung für die Transporteinrichtung 6.

Wie eingangs erwähnt, ist es erforderlich, auch im laufenden Betrieb die gesamte Vorrichtung möglichst weitgehend steril zu halten. Zu diesem Zweck werden die schematisch dargestellten ß-Strahlungseinheiten 16a, 16b, 16c, 16d verwendet. Wie in Figur 1 gezeigt, geben diese β-Strahlungseinheiten jeweils definierte Strahlungskeulen 26 ab bzw. geben definierte Strahlungsdosen an Komponenten der Vorrichtung ab.

Die dabei bestrahlten Komponenten der Vorrichtungen sind beispielsweise Führungselemente, Greifelemente, Dichtungselemente und dergleichen. Bevorzugt wird jedoch darauf geachtet, dass die Behältnisse selbst und auch das einzufüllende Getränk nicht mit der ß-Strahlung in Berührung kommen.

Die Elektronenstrahlen bzw. ß-Strahlen werden in den Strahlungseinheiten 16a - 16d im Vakuum erzeugt und beschleunigt. Anschließend treten die Strahlen durch eine 10 - 15 µm dünne Titanfolie, welche als Fenster dient, in den Atmosphärenbereich über und müssen am Ende ihrer Laufzeit noch genügend Energie besitzen, um Sporen abzutöten. Die Elektronenstrahlen können durch Materie und beispielsweise auch durch Luft sehr leicht abgebremst werden. Bei dieser Abbremsung entsteht jedoch Röntgenstrahlung, die wiederum einen erheblichen Aufwand an Abschirmung erfordert. Damit ist besonders bevorzugt ein Gehäuse 14 vorgesehen, welches besonders bevorzugt mit einer röntgenstrahlenabsorbierenden Auskleidung, beispielsweise mit einem Bleimantel, umgeben ist.

Weiterhin ist vorzugsweise eine Absaugeinrichtung 22 vorgesehen, um evtl. entstehendes Ozon abzusaugen. Bei einer anderen alternativen Variante ist es möglich, den zuständigen Behandlungsraum innerhalb des Gehäuses 14 mit Stickstoff oder Edelgas zu spülen. Das Bezugszeichen 18 bezieht sich auf eine Steuerungseinrichtung, welche die einzelnen Prozesse steuert.

Besonders bevorzugt weisen die ß-Strahlungseinheiten Austrittsfenster auf, die wenigstens 5cm, bevorzugt wenigstens 10cm und besonders bevorzugt wenigstens 15cm lang sind. Diese Längen weichen von den Längen der Austrittsfenster herkömmlicher Strahlungseinheiten ab und sind im vorliegenden Fall vorteilhaft, um das gewünschte Strahlungsprofil, welches räumlich sehr genau definiert ist, zu erzeugen. Damit weisen die Austrittsfenster die Gestalt einer Schlitzblende auf. Die Breite der Austrittsfenster liegt in einem Bereich von 0,1mm bis 1mm.

Besonders bevorzugt sind die ß-Strahlungseinheiten 16a - 16d an solchen Bereichen angeordnet, an denen im Laufe des Produktionsverfahrens keine Behältnisse vorbeigeführt werden. Beispielsweise ist an der Transporteinrichtung 5 eine β-Strahlungseinheit 16a in einem Bereich vorgesehen, in dem die Behältnisse nicht vorbeigeführt werden, da die Behältnisse in einem Übergabepunkt A aufgenommen werden und in einem Übergabepunkt B an den Füller 10 abgegeben werden. Dabei werden die Behältnisse in der Transporteinrichtung 6 entgegen des Uhrzeigersinns geführt und auf der Strecke, die entgegen des Uhrzeigersinns zwischen den Übergabepunkten B und A liegt, werden damit keine Flaschen geführt.

Damit ist es möglich, in diesem Bereich das Transportkarussell 5 zu reinigen ohne hierbei die Behältnisse selbst zu kontaminieren. Entsprechend ist auch die β-Strahlungseinheit 16b in einem Bereich zwischen dem Übergabepunkt B und dem Übergabepunkt C angeordnet, an dem keine Behältnisse geführt werden. Da sich die einzelnen Transporteinrichtungen 4, 5, 8, 9, 19 oder auch des Füllers 10 bewegen, kann durch die ß-Strahlungseinheiten eine effiziente Gesamtreinigung durchgeführt werden. Auch die Bestrahlungseinheit 16c und die Bestrahlungseinheit 16d befinden sich jeweils an Bereichen der jeweiligen Transportkarusselle 9 und 19, an welchen jeweils keine Behältnisse vorbeigeführt werden.

Das Bezugszeichen 18 bezieht sich auf eine Steuerungseinrichtung, welche sowohl die Befüllungseinrichtung 10 als auch die ß-Strahlungseinheiten steuert. Dabei ist bevorzugt der Antrieb der Befüllungseinrichtung 10 auf die Antriebe der einzelnen Transporteinheiten 4, 5, 8, 9 und 19 synchronisiert.

Das Bezugszeichen 30 bezieht sich auf eine Sterilisationseinrichtung, die zum Sterilisieren der Behältnisse selbst dient. Wie eingangs erwähnt, können der Antrieb einer Förderungseinrichtung 32, welche die Behältnisse in der Sterilisationseinrichtung 30 fördert und der Antrieb der Transporteinrichtung des Füllers 10 unabhängig voneinander gesteuert werden. Das Bezugszeichen 25 bezieht sich auf ein Gehäuse, welches die gesamte Anlage umgibt. Vorzugsweise befindet sich innerhalb dieses Gehäuses 25 eine Atmosphäre aus Stickstoff, einem Edelgas oder Sterilluft.

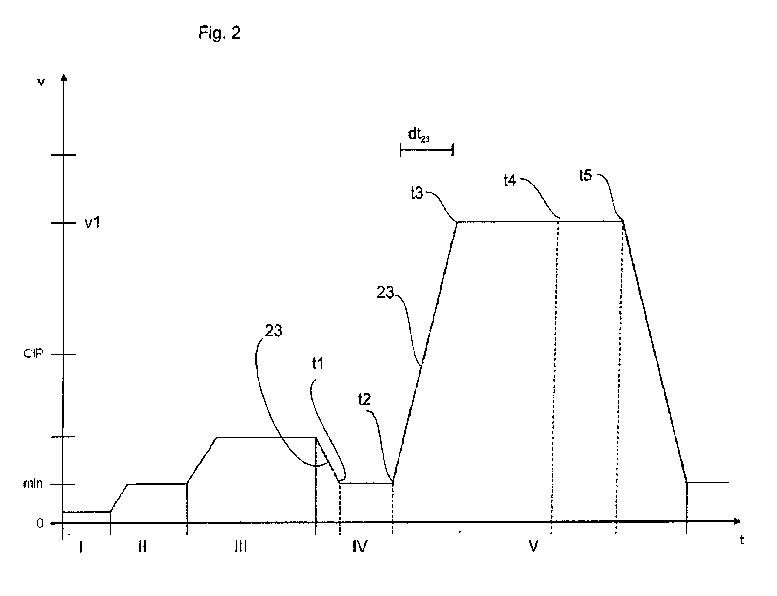

Fig. 2 zeigt schematisch und beispielhaft einen Geschwindigkeitsverlauf für eine Vorrichtung zum Befüllen von Behältnissen und insbesondere für die Transporteinrichtungen dieser Vorrichtung. Dabei ist auf der Ordinate die Zeit t aufgetragen und auf der Abszisse die Geschwindigkeit v der jeweiligen Transporteinrichtungen. In dem ersten Abschnitt I findet ein Ruhebetrieb statt. In diesem Segment könnte die Geschwindigkeit auch Null betragen. Es ist jedoch möglich, im Rahmen eines sehr langsam laufenden Betriebs beispielsweise eine Dauersterilisierung der Vorrichtung vorzunehmen. Während des Abschnitts II können beispielsweise Vorbereitungsmaßnahmen vorgenommen werden, zum Beispiel eine der Vorrichtung nachgeordnete Vorrichtung zum Verschließen der Behältnisse mit Verschlüssen befüllt werden.

Das Bezugszeichen 23 bezieht sich jeweils auf Beschleunigungs- bzw. Verzögerungsstrecken, in denen die Transportgeschwindigkeit der Vorrichtung 1 vorzugsweise linear von einer Ausgangsgeschwindigkeit auf eine Endgeschwindigkeit beschleunigt oder verzögert wird.

Im Abschnitt III wird die Vorrichtung für den eigentlichen Produktionsvorgang (Abschnitt V) vorbereitet. In diesem Zeitraum ist es möglich, die Vorrichtung bereits mit einer bestimmten ß-Strahlungsdosis zu bestrahlen. Dabei kann die Ausgangsleistung bzw. Intensität der ß-Strahlungseinheiten herabgesetzt werden, da in diesem Zeitpunkt auch die Transportgeschwindigkeit geringer ist und damit in gleicher Weise eine effiziente Sterilisierung der einzelnen Maschinenkomponenten möglich ist. In dem Zeitraum IV wird eine Synchronisierung mit einer vorgeschalteten Sterilisierungseinrichtung vorbereitet.

Dabei ist zu dem gezeigten Zeitpunkt t1 eine Flaschensperre für die Behältnisse noch geschlossen und damit gelangen noch keine Behältnisse in die Vorrichtung 1 und auch nicht in die dieser vorgeschaltete Sterilisationseinrichtung 30. Zum Zeitpunkt t2 wird die Flaschensperre automatisch geöffnet und dies bedeutet, dass nunmehr Behältnisse in die Sterilisationseinrichtung 30 gelangen können. Gleichzeitig wird bevorzugt die Geschwindigkeit der Vorrichtung 1 beschleunigt und auf die Produktionsgeschwindigkeit v1 gebracht. Dabei wird diese Beschleunigung hier mittels Servotechnik vorgenommen, wobei der Zeitraum dt23 zwischen dem Beginn des Beschleunigungsprozesses (Zeitpunkt t2) und dem Ende des Beschleunigungsprozesses (Zeitpunkt t3) der Zeit entspricht, in der die Transporteinrichtung 6 der Fülleinrichtung 10 eine komplette Umdrehung oder ganzzahlige Vielfache einer Umdrehung durchführt.

Bei dem in Figur 2 gezeigten Diagramm wird die Geschwindigkeit der Fülleinrichtung beschleunigt. Es wäre jedoch auch möglich, dass je nach Anwendung zwischen dem Zeitpunkt t2 und dem Zeitpunkt t3 auch eine Verzögerung vorgenommen wird. Ab dem Zeitpunkt t3 läuft die Vorrichtung 1 mit ihrer Produktionsgeschwindigkeit v1. In diesem Zeitraum ist die Geschwindigkeit der Fülleinrichtung auch synchron zu der Geschwindigkeit der vorgeschalteten Sterilisationseinrichtung 30. Zum Zeitpunkt t4 wird die Flaschensperre automatisch geschlossen, jedoch die Geschwindigkeit der Fülleinrichtung noch bis zu dem Zeitpunkt t5 beibehalten, um die Vorrichtung definiert zu entleeren. Auf diese Weise kann sichergestellt werden, dass alle Komponenten in dem Zeitraum dt23 genau einheitlich bestrahlt werden, zu dem sich noch Behältnisse in der Vorrichtung befinden. Ab dem Zeitpunkt t5 wird die Transporteinrichtung der Füllvorrichtung 1 wiederum verzögert.

Die Synchronisation der Transportgeschwindigkeit in dem Zeitraum dt23 erfolgt durch servomotorgesteuerte Anpassung an die Geschwindigkeit der Sterilisationseinrichtung ("on the fly").

## Patentansprüche

1. Vorrichtung (1) zum Befüllen von Behältnissen mit einem Zugang (2), durch welchen hindurch offene Behältnisse in die Vorrichtung (1) einführbar sind, mit wenigstens einer Transporteinrichtung (6), welche die Behältnisse entlang eines vorgegebenen Transportpfades befördert und mit einem Abgang (12), über den die Behältnisse aus der Vorrichtung (1) abführbar sind, wobei die Geschwindigkeit, mit der die Transporteinrichtung die Behältnisse fördert, veränderbar ist, und mit wenigstens einer Befüllungseinrichtung (10), welche die Behältnisse auf dem Transportpfad zwischen dem Zugang (4) und dem Abgang (12) wenigstens teilweise mit einem flüssigen Medium befüllt;
**dadurch gekennzeichnet, dass**
die Vorrichtung ein Gehäuse (14) aufweist und im Inneren des Gehäuses wenigstens eine Sterillsatlonseinrichtung (16a, 16b, 16c, 16d) in Form einer β- Strahlungseinheit (16a, 16b, 16c, 16d) angeordnet ist, welche Komponenten der Vorrichtung (1) sterilisiert, wobei die Intensität der wenigstens einen β- Strahlungseinheit (16a, 16b, 16c, 16d) steuerbar ist und die Vorrichtung eine Steuereinheit (18) aufweist, die derart gestaltet ist, dass sie die Intensität der wenigstens einen β-Strahlungseinheit (16a, 16b, 16c, 16d) in Abhängigkeit von der Transportgeschwindigkeit der Transporteinrichtung (6) oder einem Öffnungszustand einer stromaufwärts bezüglich der Vorrichtung vorgesehenen Sperreinrichtung für die Behältnisse (10) steuert.

2. Vorrichtung nach Anspruch 1,
**dadurch gekennzeichnet, dass**
die Komponenten aus einer Gruppe von Komponenten ausgewählt sind, welche Tansportsteme, Garnituren, Greifeinrichtungen für die Behältriisse enthält.

3. Vorrichtung nach wenigstens einem der vorangegangenen Ansprüche,
**dadurch gekennzeichnet, dass**
die wenigstens eine β-Strahlungseinheit (16a, 16b, 16c, 16d) derart beschaffen und/oder angeordnet ist, dass der aus der β- Strahlungseinheit (16a, 16b, 16c, 16d)) austretende β- Strahl während des Transports der Behältnisse nicht auf die Behältnisse gelenkt wird.

4. Vorrichtung nach wenigstens einem der vorangegangenen Ansprüche,
**dadurch gekennzeichnet, dass**
die Vorrichtung in einem Reinraum (20) angeordnet ist und dieser Reinraum mit einem Gas gefüllt ist, welches aus einer Gruppe von Gasen ausgewählt ist, welche Edelgase. Stickstoff, sterilisierte Luft enthält.

5. Vorrichtung nach wenigstens einem der vorangegangenen Ansprüche,
**dadurch gekennzeichnet, dass**
eine Vielzahl von stationär angeordneten β- Strahlungseinheiten (16a, 16b, 16c, 16d) vorgesehen ist.

6. Anlage zum Behandeln von Behältnissen mit einer Vorrichtung (1) nach wenigstens einem der vorangegangenen Ansprüche und einer stromaufwärts bezüglich dieser Vorrichtung (1) angeordneten Sterilisatidnseinrichtung (30), welche die Behältnisse sterilisiert.

7. Anlage nach Anspruch 6,
**dadurch gekennzeichnet, dass**
die Sterilisationseinrichtung (30) wenigstens eine Fördereinrichtung (32) aufweist, welche die Behältnisse durch die Sterilisationseinrichtung (30) fördert.

8. Anlage nach Anspruch 7,
**dadurch gekennzeichnet, dass**
die Antriebe der Transporteinrichtung (6) und der Fördereinrichtung (32) unabhängig voneinander steuerbar sind.

9. Anlage nach Anspruch 8,
**dadurch gekennzeichnet, dass**
der Antrieb der Transporteinrichtung auf den Antrieb der Fördereinrichtung (32) synchronisierbar ist.

10. Verfahren zum Behandeln von Behältnissen mit den Schritten,
- Zuführen von geöffneten Behältnissen in eine Vorrichtung (1) zum Befüllen von Behältnissen;
- Transport der Behältnisse und Befüllen der Behältnisse werden dieses Transports
- Abführen der gefüllten Behältnisse aus der Vorrichtung (1) zum Befüllen von Behältnissen
**dadurch gekennzeichnet, dass**
Komponenten der Vorrichtung (1) zum Befüllen von Behältnissen während des Befüllungsvorgangs durch wenigstens eine Sterilisiationseinheit sterilisiert werden und diese Sterilisatiorlseinrichtung wenigstens eine β- Strahlungseinheit (16a, 16b, 16c, 16d) aufweist und die Intensität der von der β- Strahlungseinheit (16a, 16b, 16c, 16d) abgegebenen Strahlung in Abhängigkeit von einer Transportgeschwindigkeit der Behältnisse gesteuert wird.

11. Verfahren nach Anspruch 10
**dadurch gekennzeichnet, dass**
die Vorrichtung (1) wenigstens zeitweise in einem Leerlaufmodus betrieben wird, in dem keine Behältnisse durch die Vorrichtung (1) transportiert werden, jedoch die Transportgeschwindigkeit der Vorrichtung von 0 verschieden ist, wobei in diesem Leerlaufmodus die Intensität wenigstens einer β- Strahlungseinheit (16a, 16b, 16c, 16d) reduziert wird.

12. Verfahren nach wenigstens einem der vorangegangenen Ansprüche 10 - 11
**dadurch gekennzeichnet, dass**
die Behältnisse vor dem Befüllen mit einer Sterilisationseinrichtung (30) sterilisiert werden.

13. Verfahren nach Anspruch 10-12,
**dadurch gekennzeichnet, dass**
im Rahmen eines Anfahrmodus die Fördergeschwindigkeit, mit der die Sterilisationseinrichtung (30) die Behältnisse fördert und die Transportgeschwindigkeit, mit der die Vorrichtung (1) die Behältnisse transportiert, aufeinander angepasst werden.

14. Verfahren nach Anspruch 13,
**dadurch gekennzeichnet, dass**
die Anpassung der Geschwindigkeiten während einer einer vorgegebenen Zeitspanne t erfolgt und diese Zeitspanne einem ganzzahligen Vielfachen derjenigen Zeitspanne entspricht, in der die Behältnisse vollständig in der Transporteinrichtung gefördert werden.

## Claims

1. Apparatus (1) for filling containers, comprising an entrance (2) through which open containers can be introduced into the apparatus (1), comprising at least one transport device (6) which conveys the containers along a predefined transport path, and comprising an exit (12) via which the containers can be discharged from the apparatus (1), wherein the speed at which the transport device conveys the containers is variable, and comprising at least one filling device (10) which fills the containers at least partially with a liquid medium on the transport path between the entrance (4) and the exit (12), **characterized in that** the apparatus comprises a housing (14) and arranged in the interior of the housing is at least one sterilization device (16a, 16b, 16c, 16d) in the form of a ß-radiation unit (16a, 16b, 16c, 16d) which sterilizes components of the apparatus (1), wherein the intensity of the at least one ß-radiation unit (16a, 16b, 16c, 16d) is controllable and wherein the apparatus comprises a control unit (18) which is designed such, that it controls the intensity of the at least one ß-radiation unit (16a, 16b, 16c, 16d) as a function of the transport speed of the transport device (6) or an opening state of a locking device for the containers (10) arranged upstream of the apparatus.

2. Apparatus according to claim 1, **characterized in that** the components are selected from a group of components which contains transport starwheels, fittings and gripping devices for the containers.

3. Apparatus according to at least one of the preceding claims, **characterized in that** the at least one ß-radiation unit (16a, 16b, 16c, 16d) is provided and/or arranged in such a way that the ß-ray coming from the ß-radiation unit (16a, 16b, 16c, 16d) is not directed onto the containers during transport of the containers.

4. Apparatus according to at least one of the preceding claims, **characterized in that** the apparatus is arranged in a clean chamber (20) and this clean chamber is filled with a gas selected from a group of gases which contains noble gases, nitrogen and sterilized air.

5. Apparatus according to at least one of the preceding claims, **characterized in that** a plurality of ß-radiation units (16a, 16b, 16c, 16d) are provided which are arranged in a stationary manner.

6. Installation for treating containers, comprising an apparatus (1) according to at least one of the preceding claims and a sterilization device (30) which is arranged upstream of this apparatus (1) and which sterilizes the containers.

7. Installation according to claim 6, **characterized in that** the sterilization device (30) comprises at least one conveying device (32) which conveys the containers through the sterilization device (30).

8. Installation according to claim 7, **characterized in that** the drives of the transport device (6) and of the conveying device (32) can be controlled independently of one another.

9. Installation according to claim 8, **characterized in that** the drive of the transport device can be synchronized with the drive of the conveying device (32).

10. Method for treating containers, comprising the steps:
- feeding open containers into an apparatus (1) for filling containers;
- transporting the containers and filling the containers during this transport;
- discharging the filled containers from the apparatus (1) for filling containers,
**characterized in that** components of the apparatus (1) for filling containers are sterilized by at least one sterilization unit during the filling process and this sterilization device comprises at least one ß-radiation unit (16a, 16b, 16c, 16d), and **in that** the intensity of the radiation emitted by the ß-radiation unit (16a, 16b, 16c, 16d) is controlled as a function of a transport speed of the containers.

11. Method according to -claim 10, **characterized in that** the apparatus (1) is operated at least at times in an idle mode in which no containers are transported through the apparatus (1) but the transport speed of the apparatus is other than 0, wherein in this idle mode the intensity of at least one ß-radiation unit (16a, 16b, 16c, 16d) is reduced.

12. Method according to at least one of the preceding claims 10 - 12, **characterized in that** the containers are sterilized by a sterilization device (30) prior to filling.

13. Method according to claim 10-12, **characterized in that**, during a start-up mode, the conveying speed at which the sterilization device (30) conveys the containers and the transport speed at which the apparatus (1) transports the containers are matched to one another.

14. Method according to claim 13, **characterized in that** the matching of the speeds takes place for a predefined period of time t and this period of time corresponds to a whole-number multiple of the period of time in which the containers are conveyed completely in the transport device.

## Revendications

1. Dispositif (1) pour le remplissage de récipients comprenant un accès (2) à travers lequel des récipients ouverts peuvent être introduits dans le dispositif (1), comprenant au moins un système de transport (6) qui transporte les récipients le long d'un chemin de transport prédéterminé et comprenant une sortie (12) par l'intermédiaire de laquelle les récipients peuvent être évacués du dispositif (1), dans lequel la vitesse à laquelle le système de transport achemine les récipients est modulable, et comprenant au moins un système de remplissage (10) qui remplit les récipients au moins partiellement avec un milieu liquide sur le chemin de transport entre l'accès (4) et la sortie (12),
**caractérisé en ce que**
le dispositif présente un logement (14) et au moins un système de stérilisation (16a, 16b, 16c, 16d) est disposé à l'intérieur du logement sous la forme d'une unité de rayonnement β (16a, 16b, 16c, 16d) qui stérilise les composants du dispositif (1), dans lequel l'intensité de la au moins une unité de rayonnement β (16a, 16b, 16c, 16d) est régulable et le dispositif présente une unité de commande (18) qui est conçue de manière à réguler l'intensité de la au moins une unité de rayonnement β (16a, 16b, 16c, 16d) en fonction de la vitesse de transport du système de transport (6) ou d'un état d'ouverture d'un système de blocage pour les récipients (10) prévu en amont du dispositif.

2. Dispositif selon la revendication 1,
**caractérisé en ce que**
les composants sont choisis d'un groupe de composants qui comprend des roues en étoile, des garnitures, des systèmes de préhension pour les récipients.

3. Dispositif selon au moins l'une des revendications précédentes,
**caractérisé en ce que**
la au moins une unité de rayonnement β (16a, 16b, 16c, 16d) est conçue et/ou disposée de telle sorte que le rayonnement β sortant de l'unité de rayonnement β (16a, 16b, 16c, 16d) n'est pas dirigé sur les récipients pendant le transport des récipients.

4. Dispositif selon au moins l'une des revendications précédentes,
**caractérisé en ce que**
le dispositif est disposé dans une salle blanche (20) et cette salle blanche est remplie d'un gaz qui est choisi dans le groupe des gaz qui comprend des gaz rares, de l'azote et de l'air stérilisé.

5. Dispositif selon au moins l'une des revendications précédentes,
**caractérisé en ce qu'**une multitude d'unités de rayonnement β (16a, 16b, 16c, 16d) disposées de manière stationnaire est prévue.

6. Installation pour le traitement des récipients comprenant un dispositif (1) selon au moins l'une des revendications précédentes et un système de stérilisation (30) disposé en amont de ce dispositif (1), qui stérilise les récipients.

7. Installation selon la revendication 6,
**caractérisée en ce que**
le système de stérilisation (30) présente au moins un système d'acheminement (32) qui achemine les récipients à travers le système de stérilisation (30).

8. Installation selon la revendication 7,
**caractérisée en ce que**
les entraînements du système de transport (6) et du système d'acheminement (32) sont régulables indépendamment les uns des autres.

9. Installation selon la revendication 8,
**caractérisée en ce que**
l'entraînement du système de transport peut être synchronisé sur l'entraînement du système d'acheminement (32).

10. Procédé de traitement de récipients comprenant les étapes consistant à :
- amener des récipients ouverts dans un dispositif (1) pour le remplissage des récipients ;
- transporter les récipients et remplir les récipients de ce transport
- évacuer les récipients remplis du dispositif (1) pour le remplissage des récipients
**caractérisé en ce que**
les composants du dispositif (1) pour le remplissage des récipients sont stérilisés pendant l'opération de remplissage par au moins une unité de stérilisation et ce système de stérilisation comprend au moins une unité de rayonnement β (16a, 16b, 16c, 16d) et l'intensité du rayonnement émis par l'unité de rayonnement β (16a, 16b, 16c, 16d) est régulée en fonction d'une vitesse de transport des récipients.

11. Procédé selon la revendication 10,
**caractérisé en ce que**
le dispositif (1) est exploité au moins par intermittence dans un mode de fonctionnement à vide dans lequel aucun récipient n'est transporté à travers le dispositif (1), mais la vitesse de transport du dispositif est cependant différente de 0, dans lequel, dans ce mode de fonctionnement à vide, l'intensité d'au moins une unité de rayonnement β (16a, 16b, 16c, 16d) est réduite.

12. Procédé selon au moins une des revendications précédentes 10 à 11,
**caractérisé en ce que**
les récipients sont stérilisés avec un système de stérilisation (30) avant le remplissage.

13. Procédé selon les revendications 10 à 12,
**caractérisé en ce que**
dans le cadre d'un mode de démarrage, la vitesse d'acheminement à laquelle le système de stérilisation (30) achemine les récipients et la vitesse de transport à laquelle le dispositif (1) transporte les récipients sont adaptées l'une par rapport à l'autre.

14. Procédé selon la revendication 13,
**caractérisé en ce que**
l'adaptation des vitesses s'effectue pendant un intervalle de temps prédéterminé et cet intervalle de temps correspond à un multiple entier de cet intervalle de temps dans lequel les récipients sont entièrement acheminés dans le système de transport.
